# EUROPEAN PATENT SPECIFICATION

(11) **EP 4 491 218 B1**
(45) Date of publication and mention of the grant of the patent: **11.02.2026**
(21) Application number: 24175535.4
(22) Date of filing: 13.05.2024
(51) Int. Cl.: A61N 5/06

(54) **BEAUTY FACE MASK**
SCHÖNHEITSGESICHTSMASKE
MASQUE FACIAL DE BEAUTÉ

(30) Priority: 31.07.2023 CN 202322052107 U; 14.07.2023 CN 202321866670 U
(43) Date of publication of application: 15.01.2025
(73) Proprietor: Hangzhou Ulike Technology Co., Ltd., Hangzhou, Zhejiang 310000 (CN)
(72) Inventor: Li, Guanting, Hangzhou, 310000 (CN)
(74) Representative: Murgitroyd & Company

(56) References cited:
- EP-A1- 4 112 121
- WO-A1-2013/036558
- WO-A1-2023/031619
- CN-A- 113 975 646
- US-A1- 2022 152 417
- US-A1- 2022 314 022

## Description

### TECHNICAL FIELD

Embodiments of this application relate to the field of skin care technologies, and in particular, to a beauty face mask.

### BACKGROUND

With the improvement of the quality of life, many people have different methods for skin care. Nowadays, many people choose to use a beauty instrument for skin care.

A beauty face mask is a beauty instrument that can be worn on a face. The beauty face mask mainly uses illumination, heating, massage, or the like to care for a facial skin, thereby achieving a cosmetic effect.

When a user uses an illumination-type beauty face mask to care for the facial skin, if insufficient light is emitted to the face, the cosmetic effect of the beauty face mask is greatly reduced, and then user's use experience of the product is affected.

Relevant technologies can be found in WO 2023/031619 A1, US 2022/314022 A1, US 2022/152417 A1, WO 2013/036558 A1, EP 4 112 121 A1, and CN 113975646 A. which discloses a phototherapy mask.

WO 2023/031619 A1 discloses a hpototherapy mask. Specifically, the mask comprises a multilayer construction formed from at least one flexible material so as to be capable of being manipulated to adopt a desired 3D shape profile to fit the contours of a person's face.

US 2022/314022 A1 discloses a skin care device. Specifically, a skin care device according to an embodiment of the US2022314022A1 includes an outer case having a first opening, an inner case configured to be fastened to the outer case and having a second opening corresponding to the first opening, and a light output module configured to be accommodated between the outer case and the inner case, in which the light output module includes a module body configured to form a predetermined area based on a shape of the outer case or the inner case, a module cover formed along a part of an outer part of the module body, and, between the module body and the module cover, a plurality of light sources disposed in an array form along the outer part of the module body, and in which each of the plurality of light sources is disposed to face the module body.

US 2022/152417 A1 discloses a mask device for skincare using light. Specifically, it relates to a mask device for skincare using light. The mask device for skincare using light includes: an outer mask unit; an inner mask unit located to be spaced apart from the outer mask unit; and a plurality of optical sources located between the outer mask unit and the inner mask unit and configured to output light towards the inner mask unit. The inner mask unit includes a substrate including a first surface having a plurality of recesses and a second surface located opposite to the first surface, and the optical sources are located to face the recesses, respectively.

WO 2013/036558 A1 discloses a light therapy platform system. Specifically, phototherapy systems comprise a therapeutic lamp platform for radiant lamps such as LEDs disposed in an assembly comprising a first wall to which the lamps are affixed thereto and a second wall, closer to the patient, spaced from the first wall wherein the lamps are recessed relative thereto. The second wall comprises a reflective surface facing towards a patient and a plurality of light apertures substantially aligned with the LEDs on the first wall for communicating lamp radiation from the lamps to a user. The lamps and associated circuitry are disposed between the first and second wall so that the reflective surface is relatively smooth and seamless towards the patient. The walls have a malleable rigidity for flexible adjustability relative to the user. The device is mounted to the user with a frame comprising an eyeglass frame or goggles including lenses for shielding the user's eyes from lamp radiation.

EP 4 112 121 A1 discloses a LED illuminator and complexion recovery method using same. Specifically, it relates to an LED illuminator including a face lens, and a complexion recovery method using same. Specifically, the present invention relates to: an LED illuminator including a face lens for controlling the direction and intensity of light radiated from an LED light source; and a method for alleviating skin aging and wrinkles by using same.

CN 113975646 A discloses a LED uniform light-emitting assembly conforming to human body. Specifically, it relates to a LED uniform light-emitting assembly conforming to the human body, which comprises a flexible lamp panel with array LED lamp beads; the LED lamp beads emit ultraviolet light, red light or green light, and each LED lamp bead is arranged in one cavity; a light uniformizing structure capable of achieving short-distance light uniformizing and transmitting light rays to the upper portion (lateral upper portion) after light uniformizing is arranged near the LED lamp beads in the cavity, the light uniformizing structure is in a polyhedron shape, and preferably, the light uniformizing structure is a combination of a hollow hopper structure and a pyramid structure. One flexible lamp panel comprises a plurality of array LED lamp beads, and printed circuits connected with each other are arranged in any adjacent cavities; a control structure for controlling the flexible lamp panel to emit light is additionally arranged; and a direct-current power supply for providing energy for the control structure and the flexible lamp panel is arranged. The lamp beads are arranged at the bottom and the lamp beads on the side faces, and the corresponding dodging structures are arranged. The shape of the lamp panel is matched with that of the skin, and a humanized phototherapy instrument is manufactured, is used for physical therapy and disease treatment, and is particularly suitable for diseases with curative effects generated by ultraviolet light.

### SUMMARY

In view of the shortcomings of the conventional technology, this application provides a beauty face mask, which can ensure that sufficient light can be emitted from the beauty face mask to a facial skin, to improve a cosmetic effect of the product, and ensure user's use experience of the product.

The present invention is defined by the independent claim as appended. Developments are set forth in the dependent claims.

In the foregoing technical solution of this application, each transparent area of the beauty face mask is provided with a light source module that includes at least two lamp beads, so that more lamp beads can be reasonably arranged in limited space of the beauty face mask, which can ensure that sufficient light can be emitted from each transparent area of the beauty face mask to a facial skin, to improve a cosmetic effect of the product, and ensure user's use experience of the product.

In the foregoing technical solution of this application, each transparent area of the beauty face mask is provided with the light source module, and at least some of the light source modules each include at least two lamp beads, so that more lamp beads can be reasonably arranged in limited space of the beauty face mask according to different light requirements of a facial skin in different areas, which can ensure that sufficient light can be emitted from each transparent area of the beauty face mask to the facial skin, to improve a cosmetic effect of the product, and ensure user's use experience of the product.

### BRIEF DESCRIPTION OF DRAWINGS

FIG. 1 is a schematic diagram of a structure of a beauty face mask according to an embodiment of this application;
FIG. 2 is an schematic exploded view of the structure of the beauty face mask according to the embodiment of FIG. 1, where to better display a structural feature of a substrate, a part of the substrate is removed;
FIG. 3 is a schematic diagram of a substrate arrangement manner of a light source assembly according to an embodiment of this application;
FIG. 4 is a schematic diagram of a structure of a light source assembly according to an embodiment of this application;
FIG. 5 is a sectional view of a face mask body according to an embodiment of this application;
FIG. 6 is an enlarged view of a portion D in FIG. 5;
FIG. 7 is a schematic diagram of a structure of a beauty face mask having an eye shield body according to an embodiment of this application;
FIG. 8 is a schematic diagram of a structure of a beauty face mask having an eye support assembly according to an embodiment of this application;
FIG. 9 is a schematic exploded view of a structure of an eye support assembly according to an embodiment of this application;
FIG. 10 is a schematic exploded view of a structure of a head support assembly according to an embodiment of this application;
FIG. 11A is a sectional view of a head support assembly according to an embodiment of this application; and
FIG. 11B is a sectional view of a protective layer of a head support assembly according to an embodiment of this application.

Purpose implementation, functional features, and advantages of this application are further described with reference to the embodiments and the accompanying drawings.

### DESCRIPTION OF EMBODIMENTS

In this application, the terms "dispose", "provide", and "connect" shall be understood broadly, for example, may be a fastened connection, a detachable connection, or an integral structure, or may be a mechanical connection or an electrical connection, or may be a direct connection or an indirect connection using an intermediate medium, or may be an internal connection between two apparatuses, elements, or components. A person of ordinary skill in the art may understand specific meanings of the foregoing terms in this application based on specific conditions.

An orientation or a positional relationship indicated by terms "center", "longitudinal", "transverse", "length", "width", "thickness", "up", "down", "front", "back", "left", "right", "vertical", "horizontal", "top", "bottom", "inside", "outside", "axial", "radial", "circumferential", and the like is an orientation or a positional relationship shown based on the accompanying drawings, is intended only to facilitate the description of this application and simplification of the description rather than indicating or implying that an apparatus or an element indicated needs to have a specific orientation, or be constructed and operated in a specific orientation, and therefore is not intended to be construed as a limitation to this application.

In addition, terms "first" and "second" are used only for description purposes, and cannot be understood as an indication or an implication of relative importance or as an implicit indication of a quantity of indicated technical features. Therefore, a feature limited by "first" or "second" may explicitly or implicitly include at least one such feature. In the description of this application, "a plurality of" means at least two, for example, two or three, unless otherwise specifically limited.

In addition, some of the foregoing terms may be used to indicate an orientation or positional relationship, and may also be used to indicate another meaning. For example, in some cases, the term "up" may also be used to indicate an attachment relationship or a connection relationship. A person of ordinary skill in the art may understand specific meanings of these terms in this application based on specific conditions.

To make the objectives, technical solutions, and advantages of this application clearer, the following further describes this application in detail with reference to the accompanying drawings and the embodiments. It should be understood that the specific embodiments described herein are merely used to explain this application, but are not intended to limit this application.

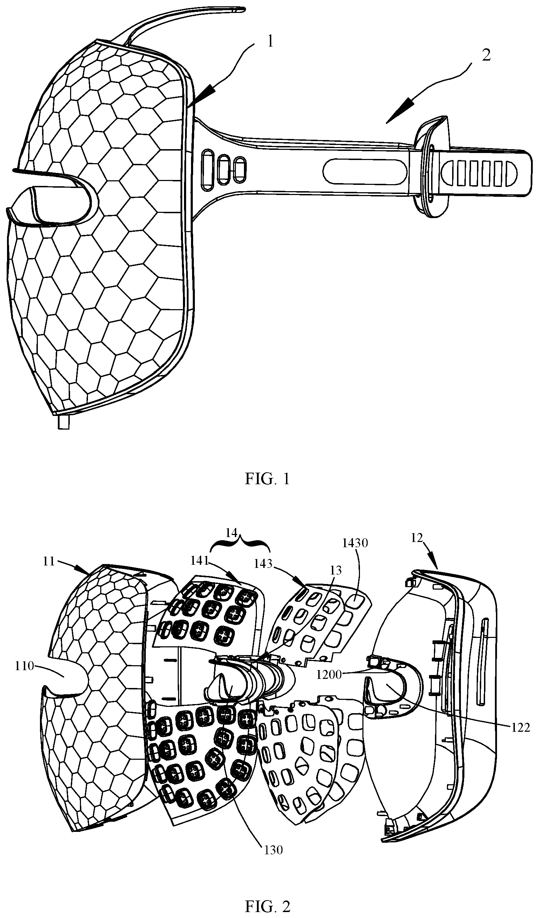

Refer to FIG. 1 to FIG. 4. An embodiment of this application provides a beauty face mask, including a face mask body 1 and a light source assembly 14. The face mask body 1 is configured to cover a face of a person, to care for a facial skin. The face mask body 1 includes an outer shell 11 and an inner shell 12 that is configured to face the face, and the outer shell 11 and the inner shell 12 enclose a mounting space. The light source assembly 14 is disposed in the mounting space, and mainly includes a substrate 1411 and several light source modules 1412 that are arranged in an array on a surface, of the substrate 1411, that faces the inner shell 12. Each light source module 1412 includes at least two lamp beads, and the inner shell 12 has several transparent areas. Each light source module 1412 corresponds to one transparent area, to emit light out of the inner shell 12 from the corresponding transparent area and then illuminate the light to the facial skin of the user.

In another embodiment of this application, that each light source module 1412 includes at least two lamp beads may be modified to the following: at least some of the light source modules each include at least two lamp beads. That is, on the basis that a light source module is disposed in each transparent area of the beauty face mask, at least two lamp beads are disposed in each of at least some of the transparent areas, so that more lamp beads can be reasonably arranged in limited space of the beauty face mask according to different light requirements of a facial skin in different areas, which can ensure that sufficient light can be emitted from each transparent area of the beauty face mask to the facial skin, to improve a cosmetic effect of the product. For example, a light-insensitive facial area has a low light requirement, and therefore, one or two lamp beads may be disposed in each transparent area corresponding to this facial area; and for a facial area with a high light requirement, more than two lamp beads may be disposed in each transparent area corresponding to this facial area, to ensure user's use experience of the product.

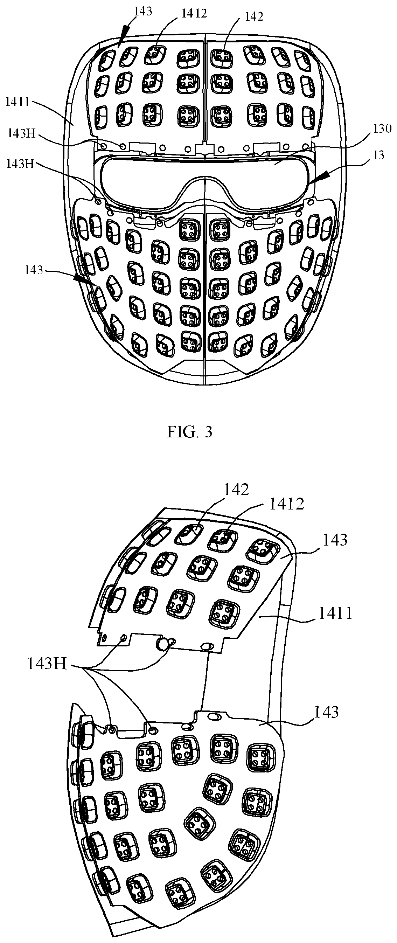

When the beauty face mask is assembled, the substrate 1411 may be first mounted on the inner shell 12 or the outer shell 11, where the light source modules 1412 face the inner shell 12, and each light source module 1412 corresponds to one transparent area of the inner shell 12; and then the outer shell 11 is disposed to cover a surface of the inner shell 12, where the inner shell 12 and the outer shell 11 form a mounting space that surrounds the light source assembly 14. When the light source modules 1412 are lit, light emitted by each light source module 1412 may pass through a corresponding transparent area of the inner shell 12 to care for a facial skin, and sufficient light is emitted from each transparent area, thereby ensuring a care effect. The several light source modules 1412 are disposed in an array on the substrate 1411. To adapt to a contour of a human face, the substrate 1411 may be arc-shaped. Therefore, that the several light source modules 1412 are disposed in an array on the substrate 1411 may be understood as that the several light source modules 1412 on the substrate 1411 have different positions and different orientations, but are proximately disposed in an array, and further, an arrangement density of the light source modules 1412 may be uniform or non-uniform, and may be set based on an actual condition (for example, a contour feature of the face). In an example, as shown in FIG. 3, the light source modules 1412 are disposed relatively densely in a middle area of the substrate 1411, and are disposed relatively dispersedly from the middle area to both sides.

In this embodiment, each transparent area of the beauty face mask is provided with a light source module 1412 that includes at least two lamp beads, so that more lamp beads can be reasonably arranged in limited space of the beauty face mask, which can ensure that sufficient light can be emitted from each transparent area of the beauty face mask to a facial skin, to improve a cosmetic effect of the product, and ensure user's use experience of the product.

Considering that the lamp bead needs to emit light at a predetermined emission angle to ensure a care effect, in an embodiment, the substrate 1411 is a flexible plate whose shape can change as required, so that each light source module 1412 is disposed in a proper position and orientation. For example, a surface, of the inner shell 12, that faces the light source assembly 14 is formed as an arc surface that arches toward the outer shell 11, and the substrate 1411 may be an arc surface matching the surface of the inner shell 12, and may be attached to a side, of the inner shell 12, that faces the outer shell 11, to maintain a good fitting effect with the surface of the inner shell 12.

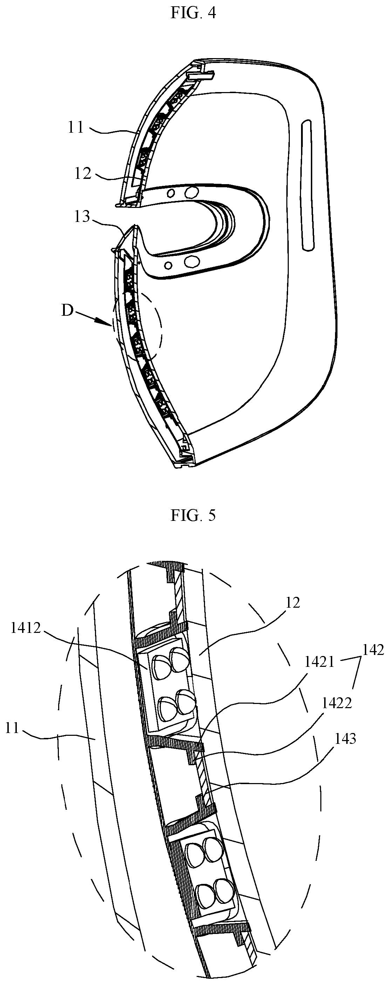

In this application, the face mask body 1 is constructed as an arc surface shape that matches the face, and correspondingly, both the inner shell 12 and the shell 11 are arc surfaces. To better adapt to the arc surface shape of the face mask body 1, as shown in FIG. 3 and FIG. 4, the substrate 1411 includes a plurality of sub-substrates, the plurality of sub-substrates are disposed adjacent to each other, and each sub-substrate corresponds to a different area on the surface of the inner shell 12. In this way, each sub-substrate may be disposed to adapt to a different surface of the inner shell 12, and the sub-substrates are spliced into a combined substrate that covers different areas of the surface of the inner shell 12. Therefore, the split spliced substrate 1411 may well adapt to the arc surface structure of the face mask body 1, and has a good fitting effect.

A light utilization ratio cannot be well ensured by only the light that is emitted by a light source to the face of a person through the inner shell 12, for example, a part of the light may be scattered, and the light cannot penetrate through the inner shell 12 and a loss is caused. In an embodiment, the light source assembly 14 includes the substrate 1411, the lamp beads, and a reflective member. The reflective member is disposed on a side, of the substrate 1411, that faces the inner shell 12, to reflect the light emitted by the lamp beads toward the inner shell 12.

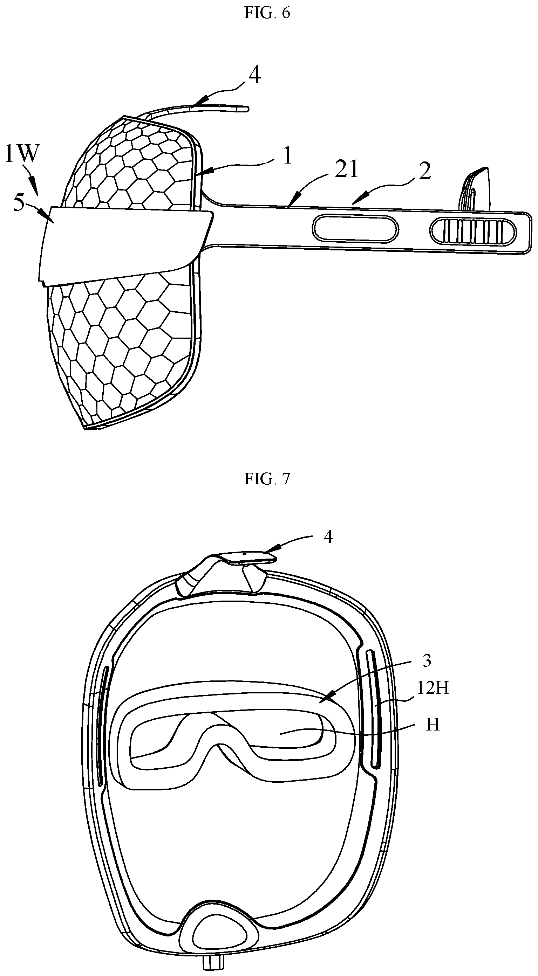

FIG. 5 is a sectional view of a face mask body according to an embodiment of this application; and FIG. 6 is an enlarged view of a portion D in FIG. 5.

Further with reference to FIG. 5 and FIG. 6, in an embodiment, the reflective member includes several reflective covers 142 disposed on a side, of the substrate 1411, that faces the inner shell 12, and each reflective cover 142 is disposed to cover the periphery of one light source module 1412, to reflect light emitted by the light source module 1412 toward the inner shell 12. For example, the reflective cover 142 is horn-shaped, one end thereof is fastened to the periphery of the light source module 1412 on the substrate 1411, and the other end thereof that faces the inner shell 12 is a free end 1421 with a large opening. The reflective cover is designed to expand outward in a horn shape, and can uniformly reflect light emitted by the light source module 1412 to a transparent area, of the inner shell 12, that directly faces the reflective cover for emission, thereby improving light emission uniformity and light coverage.

It may be understood that the reflective cover 142 may be disposed integrally with the substrate 1411, or may be disposed separately from the substrate 1411. Alternatively, at least two adjacent reflective covers 142 are connected into a whole. For example, the adjacent reflective covers 142 are connected into a whole through a connecting rib, or the adjacent reflective covers 142 are integrally formed. For example, all the reflective covers 142 are connected into a whole to form an integral structural member, to facilitate product assembly.

In some embodiments, the light source assembly 14 may further include a spacer 143 disposed on the side, of the substrate 1411, that faces the inner shell 12. In an embodiment, the spacer 143 includes several light-transmitting holes 1430 (as shown in FIG. 2) disposed in an array, and at least a part of an end, of each reflective cover 142, that faces the inner shell 12 is inserted and fastened in a corresponding light-transmitting hole 1430. In this way, the spacer 143 may be used for performing locating and auxiliary mounting on each reflective cover 142. For example, when the reflective covers 142 are disposed separately from the substrate 1411, if the reflective covers 142 need to be assembled one by one for the light source modules 1412, it is usually time-consuming. In this case, each reflective cover 142 may be mounted in a light-transmitting hole 1430 of the spacer 143 first, and then the substrate 1411 is assembled with the spacer 143 on which the reflective covers 142 are mounted, so that the reflective covers 142 and the light source modules 1412 can be quickly aligned, assembled, and fastened, thereby improving assembly efficiency.

In some embodiments, to further improve the light utilization ratio, a reflective layer is further disposed on the spacer 143, to reflect light emitted around by lamp beads to the inner shell 12, thereby implementing full utilization of light. Light is emitted from the light source modules 1412, and after the light that penetrates through the transparent areas of the inner shell 12 is reflected or diffused to the reflective layer by a facial skin of a person, the light is reflected to the facial skin by the reflective layer on the spacer 143. In this case, all areas of the inner shell 12 may be disposed as transparent areas. It may be understood that the reflective layer may be disposed on a surface, of the spacer 143, that faces the outer shell 11, or disposed on a surface, of the spacer 143, that faces the inner shell 12. It may be understood that the reflective layer is disposed on the spacer 143 to form an intensifying mirror (also referred to as a hyperlens), to implement full utilization of light, and increase the amount of light acting on the facial skin. The intensifying mirror (also referred to as a hyperlens) includes the spacer 143. The reflective layer is disposed on the spacer 143. That is, the intensifying mirror (also referred to as a hyperlens) is an assembly that includes the spacer 143 on which the reflective layer is disposed in this embodiment. It should be noted that, in terms of name, the intensifying mirror (also referred to as a hyperlens) may alternatively be referred to as a condenser, and includes the spacer 143 on which the reflective layer is disposed, to implement full utilization of light, and increase the amount of light acting on the facial skin, thereby improving utilization efficiency of light. The effect is defined as a regenerative light effect.

For example, as shown in FIG. 6, a mounting flange 1422 is disposed on an outer edge, of the reflective cover 142, that is close to the free end 1421 of the reflective cover 142. After the free end 1421 of the reflective cover 142 is inserted into the light-transmitting hole 1430 of the spacer 143, the mounting flange 1422 presses against the surface of the spacer 143, so that the reflective cover 142 may be in pluggable fit with the spacer 143. In an embodiment, the spacer 143 is of a flexible material. For example, the spacer 143 may use silica gel or rubber. The reflective cover 142 may be in interference fit with the spacer 143 to ensure a plugging effect. In addition, a shape of the flexible spacer 143 may alternatively change with an arc surface structure of the face mask body 1, to ensure a fitting effect with the inner shell 12.

Similar to the substrate 1411, in an embodiment, the spacer 143 may alternatively be designed in a separated manner, the spacer 143 includes a plurality of sub-spacers, the plurality of sub-spacers are disposed adjacent to each other, and each sub-spacer corresponds to some of the reflective covers 142. For example, the spacer 143 includes four sub-spacers, and the four sub-spacers are respectively disposed in an upper area, a lower area, a left area, and a right area of the face mask body 1, to form an arc surface that is basically consistent with a shape of the face mask body 1. For another example, the substrate 1411 includes two sub-substrates, and the two sub-substrates are arranged left and right to form an arc surface that is basically the same as the shape of the face mask body 1, and respectively correspond to an upper sub-spacer and a lower sub-spacer. It may be understood that this is only an example herein. In another embodiment, a quantity of sub-substrates included in the substrate 1411 and a quantity of sub-spacers included in the spacer 143 may be adjusted as required. For example, there are six sub-substrates and three sub-spacers.

As shown in FIG. 2, FIG. 3, and FIG. 6, in an embodiment, the spacer 143 is provided with a plurality of locating holes 143H, a plurality of locating pins 1200 are protruded on a surface, of the inner shell 12, that faces the outer shell 11, the spacer 143 is attached to the inner shell 12, and the locating pins 1200 are inserted in corresponding locating holes 143H.

The locating pins 1200 are disposed on the surface of the inner shell 12, and the spacer 143 is correspondingly provided with the locating holes 143H, so that the spacer 143 may be initially limited to the surface of the inner shell 12. After the substrate 1411 and the outer shell 11 are mounted, the spacer 143 may be reliably limited to the mounting space.

In addition, to reduce costs and reduce the weight of the beauty face mask, the reflective member may include the reflective layer, to reflect light emitted around by the lamp beads to the inner shell 12, thereby implementing full utilization of light.

In an embodiment, the reflective layer includes a first reflective layer that is disposed on the inner shell 12 and that is located outside the transparent areas, light is emitted from the light source modules 1412, the light that penetrates through the transparent areas of the inner shell 12 acts on the facial skin of the person, and after a part of the light is reflected or diffused to the first reflective layer by the facial skin of the person, the light is reflected to the facial skin by the first reflective layer. It may be understood that the first reflective layer may be disposed on a surface, of the inner shell 12, that faces the outer shell 11, or disposed on a surface, of the inner shell 12, that faces the human face.

In an embodiment, the reflective layer includes a second reflective layer disposed on a surface, of the outer shell 11, that faces the inner shell 12. Light is emitted from the light source modules 1412, a part of the light acts on the facial skin of the person after penetrating through the transparent areas of the inner shell 12, another part of the light is reflected or diffused from the substrate 1411 or the inner shell 12 to the second reflective layer, a small amount of light that penetrates through the transparent areas of the inner shell 12 is reflected or diffused to the second reflective layer by the facial skin, and the light reflected or diffused to the second reflective layer is to be reflected again, and then is emitted from the transparent areas of the inner shell 12. It may be understood that the second reflective layer further reflects again the light reflected or diffused back by the substrate 1411 or the inner shell 12. In this embodiment, the substrate 1411 may use a transparent material, so that light may be reflected again toward the face by the second reflective layer, to increase an amount of light acting on the facial skin.

In an embodiment, the reflective layer includes a third reflective layer disposed on a surface, of the substrate 1411, that faces the inner shell 12. Light is emitted from the light source modules 1412, a part of the light acts on the facial skin of the person after penetrating through the transparent areas of the inner shell 12, another part of the light is reflected or diffused from the inner shell 12 to the third reflective layer, a small amount of light that penetrates through the transparent areas of the inner shell 12 is reflected or diffused to the third reflective layer by the facial skin, and the light reflected or diffused to the third reflective layer is to be reflected again, and then is emitted from the transparent areas of the inner shell 12. It may be understood that the reflective layer is disposed on the surface, of the substrate 1411, that faces the inner shell 12 to form an intensifying mirror (also referred to as a hyperlens), to implement full utilization of light, and increase the amount of light acting on the facial skin. The intensifying mirror (also referred to as a hyperlens) includes the substrate 1411. The reflective layer is disposed on the surface, of the substrate 1411, that faces the inner shell 12. That is, the intensifying mirror (also referred to as a hyperlens) is an assembly that includes the substrate 1411 on which the reflective layer is disposed in this embodiment. It should be noted that, in terms of name, the intensifying mirror (also referred to as a hyperlens) may alternatively be referred to as a condenser, and includes the substrate 1411 on which the reflective layer is disposed, to implement full utilization of light, and increase the amount of light acting on the facial skin, thereby improving utilization efficiency of light. The effect is defined as a regenerative light effect.

It may be understood that the first reflective layer, the second reflective layer, and the third reflective layer may be reflective coatings, or may be independent reflective gaskets. For example, the first reflective layer may be a coating coated on the surface of the inner shell 12, or may be a reflective gasket attached to the surface of the inner shell 12.

In an embodiment, the light source assembly 14 includes the substrate 1411, the lamp beads, and a light guide. The light guide is disposed on a side, of the lamp beads, that faces the inner shell 12, to guide the light emitted by the lamp beads toward the inner shell 12. The light guide may be constructed as a protrusion point, a protrusion column, or the like, and plays a role in guiding the light emitted by the lamp beads to be emitted from the inner shell 12.

For example, each light source module 1412 corresponds to one light guide, and at least two adjacent light guides are connected into a whole. For example, the adjacent light guides are connected into a whole through a connecting rib, or the adjacent light guides are integrally formed. For example, all the light guides are connected into a whole to form an integral structural member.

In an embodiment, the light source assembly 14 includes both reflective covers 142 and the light guides. All reflective covers 142 of the light source modules 1412 form an integral structural member, and all the light guides also form an integral structural member. Each light source module 1412 corresponds to one reflective cover 142 and one light guide. Light emitted from the light source module 1412 is converged and reflected by the reflective cover 142, guided by the light guide, and finally emitted from the inner shell 12 to the human face for skin care. In this way, utilization of the light emitted by each light source module 1412 is maximized.

It may be understood that when the light source assembly 14 includes the reflective member or the light guides, there may be one or at least two lamp beads in each light source module 1412. The arrangement of the reflective member and the light guides in the light source assembly 14 compensates for a deficiency of an original light output, and implements an effect of ensuring a light output by improving the light utilization ratio.

In addition, a surface, of the outer shell 11, that faces away from the inner shell 12 and/or a surface, of the outer shell 11, that faces the inner shell 12 are/is formed by splicing a plurality of polygonal sub-areas adjacent to each other. That is, at least one of an inner surface or an outer surface of the outer shell 11 is formed by splicing polygonal areas. When the outer surface of the outer shell 11 is formed by splicing the polygonal areas, a unique and cool appearance effect of the beauty face mask can be improved. When the inner surface of the outer shell 11 is formed by splicing the polygonal areas, and the inner surface of the outer shell 11 has a reflective layer, an inner side of the beauty face mask may present a light-emitting surface formed by the spliced polygonal areas. For another example, each polygonal area may correspond to one light source module 1412, and a reflective layer of each polygonal area mainly reflects light emitted by one light source module 1412, thereby generating a more beautiful lighting effect.

In an embodiment, as shown in FIG. 2 and FIG. 3, the face mask body 1 further includes a window frame 13 sandwiched between the outer shell 11 and the inner shell 12, the outer shell 11 is provided with a first observation window 110, the inner shell 12 is provided with a second observation window 122, a third observation window 130 for connecting the first observation window 110 to the second observation window 122 is formed on the window frame 13, and the window frame 13 prevents light emitted by the light source assembly 14 from leaking from the mounting space to the third observation window 130, where the third observation window 130 is disposed to directly face human eyes. That is, the window frame 13 is disposed between the outer shell 11 and the inner shell 12 for blocking, and the window frame 13, the outer shell 11 and the inner shell 12 enclose a closed mounting space into which light cannot be leaked from the window frame 13.

After a user wears the beauty face mask, two eyes of the user directly face the third observation window 130, the window frame 13 sticks close to a peripheral area of the human eyes, and a human eye area directly facing the window frame 13 is basically not irradiated by light. Therefore, the user may implement functions such as normal mobile phone browsing and television watching through the third observation window 130.

As shown in FIG. 7, in an embodiment, the beauty face mask includes the face mask body 1 and a window assembly 1W, where the face mask body 1 has the third observation window 130 that is disposed to directly face human eyes; and the window assembly 1W is detachably mounted on the face mask body 1, and has an observation area corresponding to the third observation window 130.

After the beauty face mask is worn on the head of the user, the face mask body 1 covers the face of the person, and two eyes of the user directly face the third observation window 130 and the observation area of the window assembly 1W. As the window assembly 1W is detachably connected to an outer surface of the face mask body 1, in a process of performing facial care by the user by using the beauty face mask, the user may freely select a mounting state of the window assembly 1W or select a window assembly 1W with a different function, so that the beauty face mask can meet different use requirements of the user.

As shown in FIG. 7, in an embodiment, the window assembly 1W includes an eye shield body 5, the eye shield body 5 is detachably connected to a surface, of the face mask body 1, that faces away from the face of the person, and the eye shield body is disposed to cover the third observation window 130. For example, the user may freely select the mounting state of the window assembly 1W. When the eye shield body 5 is mounted on the face mask body 1, the eye shield body 5 acts as a shield in front of the third observation window 130, and the user may observe an observation area on the eye shield body 5 through the third observation window 130; and when the eye shield body 5 is removed from the face mask body 1, the user may observe a field of view outside the face mask body 1 through the third observation window 130. For example, after the user wears the beauty face mask on which the eye shield body 5 is mounted, when an observation area on the eye shield body 5 is a shading area, external light cannot pass through the eye shield body 5, and the user can enjoy an immersion scenario not disturbed by the outside. After the eye shield body 5 is removed, the user may browse a mobile phone through the third observation window 130 and obtain a field of view outside the beauty face mask. For another example, when a portion (the observation area), on the eye shield body 5, that directly faces the third observation window 130 is of a transparent material, the user may observe the field of view outside the face mask body 1 through the third observation window 130 and the observation area of the eye 5. In addition, the eye shield body 5 that has the transparent observation area can also block external dust, airflow, and the like from being blown into a human eye, thereby playing a protective role. For another example, the portion, on the eye shield body 5, that directly faces the third observation window 130 is a display screen. After the user wears the beauty face mask on which the eye shield body 5 is mounted, the user may view, through the third observation window 130, a picture on the display screen of the eye shield body 5, for example, playing a video, or experiencing a virtual reality (VR) game or an augmented reality (AR) game.

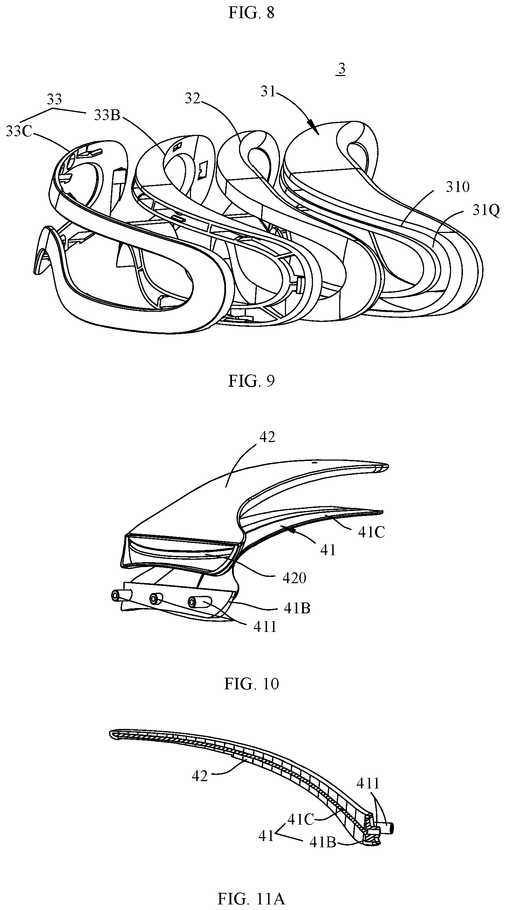

As shown in FIG. 8, in an embodiment, the window assembly 1W includes an eye support assembly 3 configured to be supported on a nose bridge, the eye support assembly 3 is detachably connected to a surface, of the face mask body 1, that faces the face of the person, the eye support assembly 3 encloses an annular eye channel H, and the eye channel H is connected to the third observation window 130.

In an embodiment, the eye support assembly 3 includes a first elastic member 31 and a second elastic member 32, where the first elastic member 31 wraps the second elastic member 32 therein, an elastic modulus of the second elastic member 32 is less than an elastic modulus of the first elastic member 31, and a side, of the first elastic member 31, that faces the face mask body 1 is connected to an inner surface of the face mask body 1.

Because the second elastic member 32 with a smaller elastic modulus is used inside the eye support assembly 3, the eye support assembly 3 may be significantly deformed after being extruded by an external force, and quickly restores its original shape after the extrusion force is removed, thereby ensuring a degree of fitting the eye support assembly 3 with the nose bridge and areas around the eyes and wearing comfort of the user when the user wears the beauty face mask. Because the first elastic member 31 with a larger elastic modulus is used outside the eye support assembly 3, the second elastic member 32 inside can be protected. The eye support member 3 has better wear resistance and a longer useful life.

In an embodiment, as shown in FIG. 9, the eye support assembly 3 further includes a mounting member 33. The mounting member 33 is disposed on a side, of the first elastic member 31, that faces the face mask body 1, and the eye support assembly 3 is detachably mounted on the inner surface of the face mask body 1 by using the mounting member 33.

The first elastic member 31 is detachably connected to the face mask body 1 by using the mounting member 33, which can ensure stable connection and convenient mounting of the both eye support assembly 3 and the face mask body 1. In addition, the mounting member 33 is disposed on the first elastic member 31 with a larger elastic modulus, which can ensure that the mounting member 33 has a relatively high combining strength with the first elastic member 31. For example, the first elastic member 31 is silica gel or rubber, and the second elastic member 32 is sponge.

For example, the first elastic member 31 includes an accommodating cavity 31Q with an open end and a third turning edge 310 disposed at the open end of the accommodating cavity 31Q, where the third turning edge 310 extends to the inside of the accommodating cavity 31Q. The mounting member 33 includes a first sub-mounting member 33B and a second sub-mounting member 33C, where the first sub-mounting member 33B is disposed in the accommodating cavity 31Q, the second sub-mounting member 33C is disposed outside the accommodating cavity 31Q, the second sub-mounting member 33C is detachably connected to the first sub-mounting member 33B, the third turning edge 310 is pressed between the first sub-mounting member 33B and the second sub-mounting member 33C, and the second sub-mounting member 33C is magnetically attracted to the inner surface of the face mask body 1.

In this way, even if the first elastic member 31 of the eye support assembly 3 is pulled, the first elastic member 31 is not easily detached from the face mask body 1 under the pressing of the mounting member 33, thereby ensuring fastening reliability of the first elastic member 31.

It may be understood that the first elastic member 31 and the second elastic member 32 may be combined together and the first sub-mounting member 33B of the mounting member 33 and the first elastic member 31 and/or the second elastic member 32 may be combined together, for example, combined together by using a glue, or the mounting member 33, the first elastic member 31, and the second elastic member 32 are combined together.

For example, the first sub-mounting member 33B may be interlocked with the second sub-mounting member 33C. Specifically, when the eye support assembly 3 is mounted, the assembled first elastic member 31, second elastic member 32, and first sub-mounting member 33B are interlocked with the second sub-mounting member 33C first, so that the first elastic member 31, the second elastic member 32, and the first sub-mounting member 33B form an integral structure with the second sub-mounting member 33C; and then the integral structure is mounted to the inner shell 12 of the face mask body 1.

With reference to FIG. 8 and FIG. 10, in an embodiment, the beauty face mask further includes a head support assembly 4, and the head support assembly 4 is connected to the top of the face mask body 1 and is configured to lap over the head of the user.

For example, a fastening belt assembly 2 includes a belt body, and two ends of the belt body are respectively fastened to left and right sides of the fastening belt assembly 1. When the user wears the face mask, the belt body of the fastening belt assembly 2 is enclosed into a closed loop, and the fastening belt assembly 2 is sleeved on the head of the user, so that the fastening belt assembly 1 is located in a position that directly faces the face.

As shown in FIG. 7 and FIG. 8, in an embodiment, two opposite sides on the inner shell 12 each are provided with a mounting hole 12H connected to the mounting space, the fastening belt assembly 2 includes the belt body 21 and two connectors (not shown in the figures) respectively disposed at two ends of the belt body 21, the two ends of the belt body 21 are respectively inserted in the two mounting holes 12H, and the connectors are fastened in the mounting space.

The inner shell of the face mask body is provided with the mounting holes 12H, and after the face mask is assembled, the two ends of the belt body 21 are respectively inserted in the two mounting holes 12H, and are fastened, by using respective connectors, in the mounting space enclosed by the outer shell 11 and the inner shell 12, so that the connectors of the belt body 21 are hidden in the face mask body 1, and the connectors at the two ends of the belt body 21 cannot be observed from the outside by the user. Therefore, a connection portion between the fastening belt assembly 2 and the face mask body 1 can be prevented from being exposed outside, thereby improving an aesthetic effect of the face mask.

During wearing, the fastening belt assembly 2 is sleeved on the head of the user, and plays a main fixing role. The head support assembly 4 is lapped over the head of the user, and plays an auxiliary positioning and supporting role.

Considering that the head support assembly 4 needs to have specific rigidity and flexibility, to ensure a lapping load-bearing function of the head support assembly 4, and avoid wearing discomfort caused due to a hard contact between the head support assembly and the head of the user or tissue damage caused due to a long-term contact with a scalp, as shown in FIG. 8 and FIG. 10 to FIG. 11B, the head support assembly 4 includes a rigid member 41 and a soft protective layer 42, one end of the rigid member 41 is detachably connected to the face mask body 1, the other end extends away from the outer surface of the face mask body 1 to form a lapping portion, and the protective layer 42 wraps the rigid member 41 therein.

When the head support assembly 4 is lapped over the head, the internal rigid member 41 can ensure that the head support assembly 4 has sufficient strength, to avoid easy deformation of an overall contour of the head support assembly 4. The soft protective layer 42 at an outer layer is in direct contact with human body tissues, and may have a large contact area with the head, to improve a fitting effect, thereby improving wearing comfort, and protecting the head of the user to some extent.

For example, an extension portion 41C is arc-shaped, and may be adapted to a head shape of a person, to achieve a better fitting effect. Optionally, the rigid member 41 and the protective layer 42 are integrally formed, or the rigid member 41 and the protective layer 42 are fastened together in a manner such as glue bonding.

As shown in FIG. 10 and FIG. 11A, in an embodiment, a first cavity 420 is formed inside the protective layer 42, and at least a part of the rigid member 41 is disposed in the first cavity 420. Specifically, the rigid member 41 includes a blocky mounting portion 41B and the extension portion 41C connected to the back of the mounting portion 41B, where the extension portion 41C is obliquely disposed in a direction away from the face mask body 1, and the mounting portion 41B is detachably connected to the face mask body 1, so that the entire head support assembly 4 is fastened to the face mask body 1.

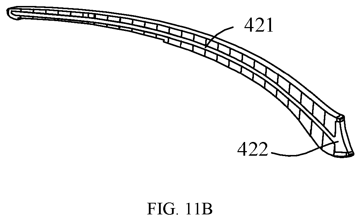

As shown in FIG. 11B, in an embodiment, the first cavity 420 includes a first sub-space 421 and a second sub-space 422 that are connected to each other, the extension portion 41C is embedded in the first sub-space 421, and the mounting portion 41B is embedded in the second sub-space 422. In this way, the extension portion 41C is attached to an inner wall forming the first sub-space 421, and the mounting portion 41B is attached to an inner wall forming the second sub-space 422. For example, the extension portion 41C and the mounting portion 41B are bonded together with the protective layer 42 by using a glue, or the rigid member 41 and the protective layer 42 are integrally formed through double-shot molding.

The head support assembly 4 is lapped over the top of the head of the user, the eye support assembly 3 is used to assist in supporting the face mask body 1 on the nose bridge of the user, and the fastening belt assembly 2 is used to fasten the face mask body 1 to the head of the user, so that the face mask body 1 is firmly and accurately fastened to the head of the user at a plurality of parts. The fastening belt assembly 2, the eye support assembly 3, and the head support assembly 4 cooperate with each other to disperse the gravity of the face mask on a plurality of parts of the head of the user, to implement good wearing comfort. In addition, the face mask does not easily shift during wearing, which helps ensure a care effect.

The foregoing descriptions are merely specific implementations of this application. It should be noted that a person of ordinary skill in the art may make some improvements and ornaments without departing from the principle of this application. These improvements and ornaments shall also be considered as being within the protection scope of this application.

## Claims

1. A beauty face mask, comprising
a face mask body (1), configured to cover a face of a person, and comprising an outer shell (11) and an inner shell (12) that is configured to face the face, wherein the outer shell (11) and the inner shell (12) enclose a mounting space; and
a light source assembly (14), disposed in the mounting space, and comprising a substrate (1411), several light source modules (1412), and a reflective member, wherein the several light source modules (1412) are arranged in an array on a surface of the substrate (1411) that faces the inner shell (12), and the reflective member is to reflect light emitted by the light source modules (1412) toward the inner shell (12); and
at least some of the light source modules (1412) comprises at least two lamp beads, the inner shell (12) has several transparent areas, and each of the light source modules (1412) corresponds to one of the transparent areas, to emit light out of the inner shell (12) from the corresponding transparent area;
wherein the reflective member comprises a first reflective layer, a second reflective layer, and a third reflective layer; the first reflective layer is disposed on a surface of the inner shell (12) and is located outside the transparent areas; the second reflective layer is disposed on a surface, of the outer shell (11), that faces the inner shell (12); and the third reflective layer is disposed on the surface of the substrate (1411) that faces the inner shell (12);
wherein the substrate (1411) comprises a plurality of sub-substrates, the plurality of sub-substrates are disposed adjacent to each other, and each of the sub-substrates corresponds to a different area on the surface of the inner shell (12), such that the sub-substrates are spliced into a combined substrate that covers different areas of the surface of the inner shell (12).

2. The beauty face mask according to claim 1, wherein the substrate (1411) is a flexible plate; and/or a surface of the inner shell (12) that faces the light source assembly (14) is formed as an arc surface that arches toward the outer shell (11), and the substrate (1411) is an arc surface, and is attached to a side of the inner shell (12) that faces the outer shell (11).

3. The beauty face mask according to claim 1, wherein
at least one of the first reflective layer, the second reflective layer, and the third reflective layer is a reflective coating or a reflective gasket.

4. The beauty face mask according to claim 1, wherein a surface of the outer shell (11) that faces away from the inner shell (12) is formed by splicing a plurality of polygonal sub-areas adjacent to each other, and/or a surface of the outer shell (11) that faces the inner shell (12) is formed by splicing a plurality of polygonal sub-areas adjacent to each other.

5. The beauty face mask according to any one of claims 1 to 4, wherein the face mask body (1) further comprises a window frame (13) sandwiched between the outer shell (11) and the inner shell (12), the outer shell (11) is provided with a first observation window (110), the inner shell (12) is provided with a second observation window (122), a third observation window (130) for connecting the first observation window (110) to the second observation window (122) is formed on the window frame (13), and the window frame (13) prevents light emitted by the light source assembly (14) from leaking from the mounting space to the third observation window (130).

6. The beauty face mask according to claim 5, further comprising:
a window assembly (1W), wherein the window assembly (1W) is detachably mounted on the face mask body (1) and has an observation area corresponding to the third observation window (130).

7. The beauty face mask according to claim 6, wherein the window assembly (1W) comprises an eye shield body (5), the eye shield body (5) is detachably connected to a surface of the face mask body (1) that faces away from the face of the person, and the eye shield body (5) is disposed to cover the third observation window (130).

8. The beauty face mask according to claim 6, wherein the window assembly (1W) comprises an eye support assembly (3) configured to be supported on a nose bridge, the eye support assembly (3) is detachably connected to a surface of the face mask body (1) that faces the face of the person, the eye support assembly (3) encloses an annular eye channel (H), and the eye channel (H) is connected to the third observation window (130).

9. The beauty face mask according to claim 1, wherein the reflective member comprises reflective covers (142), at least two adjacent reflective covers (142) are connected into a whole, the reflective covers (142) are disposed on a side, of the substrate (1411), that faces the inner shell (12), and each of the reflective cover (142) is disposed to cover periphery of one light source module (1412), to reflect light emitted by the light source module (1412) toward the inner shell (12).

10. The beauty face mask according to claim 1, wherein the light source assembly (14) further comprises a spacer (143) disposed on the side of the substrate (1411) that faces the inner shell (12); and
the spacer (143) comprises several light-transmitting holes (1430) disposed in an array, and at least a part of an end of each of the reflective covers (142) that faces the inner shell (12) is inserted and fastened in a corresponding light-transmitting hole (1430).

11. The beauty face mask according to claim 10, wherein the spacer (143) is provided with a plurality of locating holes (143H), a plurality of locating pins (1200) are protruded on a surface of the inner shell (12) that faces the outer shell (11), the spacer (143) is attached to the inner shell (12), and the locating pins (1200) are inserted in corresponding locating holes (143H).

12. The beauty face mask according to claim 1, further comprising a head support assembly (4), connected to the top of the face mask body (1), and configured to lap over a head of the person,
wherein the head support assembly (4) comprises a rigid member (41) and a soft protective layer (42), one end of the rigid member (41) is detachably connected to the face mask body (1), the other end of the rigid member (41) extends away from an outer surface of the face mask body (1) to form a lapping portion, and the protective layer (42) wraps the rigid member (41) therein.

13. The beauty face mask according to claim 12, wherein the head support assembly (4) further comprises a first cavity (420) that is formed inside the protective layer (42), and at least a part of the rigid member (41) is disposed in the first cavity (420).

14. The beauty face mask according to claim 13, wherein the rigid member (41) comprises a mounting portion (41B) and an extension portion (41C) connected to a back of the mounting portion (41B),
wherein the extension portion (41C) is obliquely disposed in a direction away from the face mask body (1), and the mounting portion (41B) is detachably connected to the face mask body (1).

## Patentansprüche

1. Eine Gesichtsmaske, umfassend:
einen Gesichtsmaskenkörper (1), der so konfiguriert ist, dass er das Gesicht einer Person bedeckt, und eine Außenhülle (11) und eine Innenhülle (12) umfasst, die so konfiguriert ist, dass sie dem Gesicht zugewandt ist, wobei die Außenhülle (11) und die Innenhülle (12) einen Befestigungsraum umschließen; und
eine Lichtquellenanordnung (14), die in dem Befestigungsraum angeordnet ist und ein Substrat (1411), mehrere Lichtquellenmodule (1412) und ein reflektierendes Element umfasst, wobei die mehreren Lichtquellenmodule (1412) in einer Anordnung auf einer Oberfläche des Substrats (1411) angeordnet sind, die der Innenhülle (12) zugewandt ist, und das reflektierende Element dazu dient, von den Lichtquellenmodulen (1412) emittiertes Licht in Richtung der Innenhülle (12) zu reflektieren; und
mindestens einige der Lichtquellenmodule (1412) mindestens zwei Lampenperlen umfassen, die Innenhülle (12) mehrere transparente Bereiche aufweist und jedes der Lichtquellenmodule (1412) einem der transparenten Bereiche entspricht, um Licht aus der Innenhülle (12) aus dem entsprechenden transparenten Bereich zu emittieren;
wobei das reflektierende Element eine erste reflektierende Schicht, eine zweite reflektierende Schicht und eine dritte reflektierende Schicht umfasst; die erste reflektierende Schicht auf einer Oberfläche der Innenhülle (12) angeordnet ist und sich außerhalb der transparenten Bereiche befindet; die zweite reflektierende Schicht auf einer Oberfläche der Außenhülle (11) angeordnet ist, die der Innenhülle (12) zugewandt ist; und die dritte reflektierende Schicht auf der Oberfläche des Substrats (1411) angeordnet ist, die der Innenhülle (12) zugewandt ist;
wobei das Substrat (1411) eine Vielzahl von Untersubstraten umfasst, die Vielzahl von Untersubstraten nebeneinander angeordnet ist und jedes der Untersubstrate einem anderen Bereich auf der Oberfläche der Innenhülle (12) entspricht, so dass die Untersubstrate zu einem kombinierten Substrat zusammengefügt sind, das verschiedene Bereiche der Oberfläche der Innenhülle (12) bedeckt.

2. Die Schönheitsgesichtsmaske gemäß Anspruch 1, wobei das Substrat (1411) eine flexible Platte ist; und/oder eine Oberfläche der Innenhülle (12), die der Lichtquellenanordnung (14) zugewandt ist, als eine zum Außenmantel (11) hin gewölbte Bogenfläche ausgebildet ist, und das Substrat (1411) eine Bogenfläche ist und an einer Seite der Innenhülle (12) angebracht ist, die dem Außenmantel (11) zugewandt ist.

3. Die Schönheitsgesichtsmaske gemäß Anspruch 1, wobei
mindestens eine der ersten reflektierenden Schicht, der zweiten reflektierenden Schicht und der dritten reflektierenden Schicht eine reflektierende Beschichtung oder eine reflektierende Dichtung ist.

4. Die Schönheitsgesichtsmaske nach Anspruch 1, wobei eine Oberfläche der Außenhülle (11), die von der Innenhülle (12) weg zeigt, durch Spleißen einer Vielzahl von aneinander angrenzenden polygonalen Teilbereichen gebildet ist und/oder eine Oberfläche der Außenhülle (11), die der Innenhülle (12) zugewandt ist, durch Spleißen einer Vielzahl von aneinander angrenzenden polygonalen Teilbereichen gebildet ist.

5. Schönheitsgesichtsmaske nach einem der Ansprüche 1 bis 4, wobei der Gesichtsmaskenkörper (1) ferner einen Fensterrahmen (13) umfasst, der zwischen der Außenhülle (11) und der Innenhülle (12) angeordnet ist, wobei die Außenhülle (11) mit einem ersten Sichtfenster (110) versehen ist, die Innenhülle (12) mit einem zweiten Sichtfenster (122) versehen ist, wobei ein drittes Sichtfenster (130) zum Verbinden des ersten Sichtfensters (110) mit dem zweiten Sichtfenster (122) an dem Fensterrahmen (13) ausgebildet ist und der Fensterrahmen (13) verhindert, dass von der Lichtquellenanordnung (14) emittiertes Licht aus dem Befestigungsraum zum dritten Sichtfenster (130) austritt.

6. Die Schönheitsgesichtsmaske gemäß Anspruch 5, die ferner umfasst:
eine Fensteranordnung (1W), wobei die Fensteranordnung (1W) abnehmbar an dem Gesichtsmaskenkörper (1) angebracht ist und einen Beobachtungsbereich aufweist, der dem dritten Sichtfenster (130) entspricht.

7. Die Schönheitsgesichtsmaske gemäß Anspruch 6, wobei die Fensteranordnung (1W) einen Augenschutzkörper (5) umfasst, der Augenschutzkörper (5) abnehmbar mit einer Oberfläche des Gesichtsmaskenkörpers (1) verbunden ist, die vom Gesicht der Person weg zeigt, und der Augenschutzkörper (5) so angeordnet ist, dass er das dritte Sichtfenster (130) abdeckt.

8. Schönheitsgesichtsmaske nach Anspruch 6, wobei die Fensteranordnung (1W) eine Augenstützanordnung (3) umfasst, die so konfiguriert ist, dass sie auf einem Nasenrücken abgestützt wird, wobei die Augenstützanordnung (3) abnehmbar mit einer Oberfläche des Gesichtsmaskenkörpers (1) verbunden ist, die dem Gesicht der Person zugewandt ist, wobei die Augenstützanordnung (3) einen ringförmigen Augenkanal (H) umschließt und der Augenkanal (H) mit dem dritten Sichtfenster (130) verbunden ist.

9. Die Schönheitsgesichtsmaske gemäß Anspruch 1, wobei das reflektierende Element reflektierende Abdeckungen (142) umfasst, mindestens zwei benachbarte reflektierende Abdeckungen (142) zu einem Ganzen verbunden sind, die reflektierenden Abdeckungen (142) auf einer Seite des Substrats (1411) angeordnet sind, die der Innenhülle (12) zugewandt ist, und jede der reflektierenden Abdeckungen (142) so angeordnet ist, dass sie den Umfang eines Lichtquellenmoduls (1412) abdeckt, um das von dem Lichtquellenmodul (1412) emittierte Licht in Richtung der Innenhülle (12) zu reflektieren.

10. Die Schönheitsgesichtsmaske gemäß Anspruch 1, wobei die Lichtquellenanordnung (14) ferner einen Abstandshalter (143) umfasst, der auf der Seite des Substrats (1411) angeordnet ist, die der Innenhülle (12) zugewandt ist; und
der Abstandshalter (143) mehrere lichtdurchlässige Löcher (1430) umfasst, die in einer Anordnung angeordnet sind, und mindestens ein Teil eines Endes jeder der reflektierenden Abdeckungen (142), das der Innenhülle (12) zugewandt ist, in ein entsprechendes lichtdurchlässiges Loch (1430) eingeführt und darin befestigt ist.

11. Schönheitsgesichtsmaske nach Anspruch 10, wobei der Abstandshalter (143) mit einer Vielzahl von Positionierungslöchern (143H) versehen ist, eine Vielzahl von Positionierungsstiften (1200) auf einer Oberfläche der Innenhülle (12) hervorstehen, die der Außenhülle (11) zugewandt ist, der Abstandshalter (143) an der Innenhülle (12) befestigt ist und die Positionierungsstifte (1200) in entsprechende Positionierungslöchern (143H) eingeführt sind.

12. Die Schönheitsgesichtsmaske gemäß Anspruch 1, die ferner eine Kopfstützenanordnung (4) umfasst, die mit der Oberseite des Gesichtsmaskenkörpers (1) verbunden ist und so konfiguriert ist, dass sie über den Kopf der Person gelegt werden kann,
wobei die Kopfstützenanordnung (4) ein starres Element (41) und eine weiche Schutzschicht (42) umfasst, ein Ende des starren Elements (41) lösbar mit dem Gesichtsmaskenkörper (1) verbunden ist, das andere Ende des starren Elements (41) sich von einer Außenfläche des Gesichtsmaskenkörpers (1) weg erstreckt, um einen überlappenden Abschnitt zu bilden, und die Schutzschicht (42) das starre Element (41) darin umhüllt.

13. Schönheitsgesichtsmaske nach Anspruch 12, wobei die Kopfstützenanordnung (4) ferner einen ersten Hohlraum (420) umfasst, der innerhalb der Schutzschicht (42) ausgebildet ist, und wobei mindestens ein Teil des starren Elements (41) in dem ersten Hohlraum (420) angeordnet ist.

14. Schönheitsgesichtsmaske nach Anspruch 13, wobei das starre Element (41) einen Befestigungsabschnitt (41B) und einen Verlängerungsabschnitt (41C) umfasst, der mit einer Rückseite des Befestigungsabschnitts (41B) verbunden ist,
wobei der Verlängerungsabschnitt (41C) schräg in einer Richtung weg vom Gesichtsmaskenkörper (1) angeordnet ist und der Befestigungsabschnitt (41B) lösbar mit dem Gesichtsmaskenkörper (1) verbunden ist.

## Revendications

1. Masque facial de beauté, comprenant :
un corps de masque facial (1), configuré pour recouvrir le visage d'une personne, et comprenant une coque extérieure (11) et une coque intérieure (12) qui est configurée pour faire face au visage, dans lequel la coque extérieure (11) et la coque intérieure (12) renferment un espace de montage ; et
un ensemble de sources lumineuses (14), disposé dans l'espace de montage, et comprenant un substrat (1411), plusieurs modules de source lumineuse (1412) et un élément réfléchissant, dans lequel les plusieurs modules de source lumineuse (1412) sont disposés en réseau sur une surface du substrat (1411) qui fait face à la coque intérieure (12), et l'élément réfléchissant sert à réfléchir la lumière émise par les modules de source lumineuse (1412) vers la coque intérieure (12) ; et
au moins certains des modules de source lumineuse (1412) comprennent au moins deux billes de lampe, la coque intérieure (12) comporte plusieurs zones transparentes, et chacun des modules de source lumineuse (1412) correspond à l'une des zones transparentes, afin d'émettre de la lumière hors de la coque intérieure (12) à partir de la zone transparente correspondante ;
dans lequel l'élément réfléchissant comprend une première couche réfléchissante, une deuxième couche réfléchissante et une troisième couche réfléchissante ; la première couche réfléchissante est disposée sur une surface de la coque intérieure (12) et est située à l'extérieur des zones transparentes ; la deuxième couche réfléchissante est disposée sur une surface de la coque extérieure (11) qui fait face à la coque intérieure (12) ; et la troisième couche réfléchissante est disposée sur la surface du substrat (1411) qui fait face à la coque intérieure (12) ;
dans lequel le substrat (1411) comprend une pluralité de sous-substrats, la pluralité de sous-substrats sont disposés de manière adjacente les uns aux autres, et chacun des sous-substrats correspond à une zone différente sur la surface de la coque interne (12), de telle sorte que les sous-substrats sont assemblés en un substrat combiné qui recouvre différentes zones de la surface de la coque interne (12).

2. Masque de beauté selon la revendication 1, dans lequel le substrat (1411) est une plaque flexible ; et/ou une surface de la coque intérieure (12) qui fait face à l'ensemble de source lumineuse (14) est formée comme une surface arquée qui s'incurve vers la coque extérieure (11), et le substrat (1411) est une surface arquée et est fixé à un côté de la coque intérieure (12) qui fait face à la coque extérieure (11).

3. Masque de beauté selon la revendication 1, dans lequel
au moins l'une parmi la première couche réfléchissante, la deuxième couche réfléchissante et la troisième couche réfléchissante est un revêtement réfléchissant ou un joint réfléchissant.

4. Masque de beauté selon la revendication 1, dans lequel une surface de la coque extérieure (11) qui est opposée à la coque intérieure (12) est formée en assemblant une pluralité de sous-zones polygonales adjacentes les unes aux autres, et/ou une surface de la coque extérieure (11) qui est tournée vers la coque intérieure (12) est formée en assemblant une pluralité de sous-zones polygonales adjacentes les unes aux autres.

5. Masque de beauté selon l'une quelconque des revendications 1 à 4, dans lequel le corps du masque (1) comprend en outre un cadre de fenêtre (13) pris en sandwich entre la coque extérieure (11) et la coque intérieure (12), la coque extérieure (11) est pourvue d'une première fenêtre (110), la coque intérieure (12) est pourvue d'une deuxième fenêtre (122), une troisième fenêtre (130) pour relier la première fenêtre (110) à la deuxième fenêtre (122) est formée sur le cadre de fenêtre (13), et le cadre de fenêtre (13) empêche la lumière émise par l'ensemble de source lumineuse (14) de s'échapper de l'espace de montage vers la troisième fenêtre (130).

6. Masque de beauté selon la revendication 5, comprenant en outre :
un ensemble de fenêtre (1W), dans lequel l'ensemble de fenêtre (1W) est monté de manière amovible sur le corps du masque (1) et comporte une zone d'observation correspondant à la troisième fenêtre (130).

7. Masque facial esthétique selon la revendication 6, dans lequel l'ensemble fenêtre (1W) comprend un corps de protection oculaire (5), le corps de protection oculaire (5) est relié de manière amovible à une surface du corps du masque facial (1) qui est opposée au visage de la personne, et le corps de protection oculaire (5) est disposé de manière à recouvrir la troisième fenêtre (130).

8. Masque facial de beauté selon la revendication 6, dans lequel l'ensemble fenêtre (1W) comprend un ensemble de support oculaire (3) configuré pour être supporté sur un pont nasal, l'ensemble de support oculaire (3) est relié de manière amovible à une surface du corps du masque facial (1) qui fait face au visage de la personne, l'ensemble de support oculaire (3) renferme un canal oculaire annulaire (H), et le canal oculaire (H) est relié à la troisième fenêtre (130).

9. Masque facial de beauté selon la revendication 1, dans lequel l'élément réfléchissant comprend des couvercles réfléchissants (142), au moins deux couvercles réfléchissants adjacents (142) sont reliés pour former un tout, les couvercles réfléchissants (142) sont disposés sur un côté du substrat (1411) qui fait face à la coque intérieure (12), et chacun des couvercles réfléchissants (142) est disposé de manière à recouvrir la périphérie d'un module de source lumineuse (1412), afin de réfléchir la lumière émise par le module de source lumineuse (1412) vers la coque interne (12).

10. Masque de beauté selon la revendication 1, dans lequel l'ensemble de source lumineuse (14) comprend en outre une entretoise (143) disposée sur le côté du substrat (1411) qui fait face à la coque intérieure (12) ; et
l'entretoise (143) comprend plusieurs trous transmettant la lumière (1430) disposés en réseau, et au moins une partie d'une extrémité de chacun des couvercles réfléchissants (142) qui fait face à la coque interne (12) est insérée et fixée dans un trou transmettant la lumière correspondant (1430).

11. Masque de beauté selon la revendication 10, dans lequel l'entretoise (143) est pourvue d'une pluralité de trous de positionnement (143H), une pluralité de broches de positionnement (1200) font saillie sur une surface de la coque intérieure (12) qui fait face à la coque extérieure (11), l'entretoise (143) est fixée à la coque intérieure (12), et les broches de positionnement (1200) sont insérées dans des trous de positionnement correspondants (143H).

12. Masque de beauté selon la revendication 1, comprenant en outre un ensemble de support de tête (4), relié à la partie supérieure du corps du masque (1) et configuré pour recouvrir la tête de la personne,
dans lequel l'ensemble de support de tête (4) comprend un élément rigide (41) et une couche protectrice souple (42), une extrémité de l'élément rigide (41) est reliée de manière amovible au corps du masque facial (1), l'autre extrémité de l'élément rigide (41) s'étend à partir d'une surface extérieure du corps du masque facial (1) pour former une partie recouvrante, et la couche protectrice (42) enveloppe l'élément rigide (41).

13. Masque facial de beauté selon la revendication 12, dans lequel l'ensemble de support de tête (4) comprend en outre une première cavité (420) qui est formée à l'intérieur de la couche protectrice (42), et au moins une partie de l'élément rigide (41) est disposée dans la première cavité (420).

14. Masque facial de beauté selon la revendication 13, dans lequel l'élément rigide (41) comprend une partie de montage (41B) et une partie d'extension (41C) reliée à l'arrière de la partie de montage (41B),
dans lequel la partie d'extension (41C) est disposée obliquement dans une direction s'éloignant du corps du masque facial (1), et la partie de montage (41B) est reliée de manière amovible au corps du masque facial (1).
